# FASCICULE DE BREVET EUROPEEN

(11) **EP 0 453 365 B1**
(45) Date de publication et mention de la délivrance du brevet: **30.11.1994**
(21) Numéro de dépôt: 91401025.1
(22) Date de dépôt: 18.04.1991
(51) Int. Cl.: C07D 295/096

(54) **Procédé de préparation de la 1-(2,3,4-triméthoxybenzyl) pipérazine par amination réductive**
Verfahren zur Herstellung von 1-(2,3,4-Trimethoxybenzyl)-Piperazin durch reduktive Aminierung
Process for the preparation of 1-(2,3,4-trimethoxybenzyl) piperazine by reductive amination

(30) Priorité: 20.04.1990 FR 9005027
(43) Date de publication de la demande: 23.10.1991
(73) Titulaire: ADIR ET COMPAGNIE, 92415 Courbevoie Cédex (FR)
(72) Inventeur: Souvie, Jean-Claude, F-76210 Bolbec (FR)
(74) Mandataire: Reverbori, Marcelle

(56) Documents cités:
- FR-A- 1 302 958
- FR-A- 2 493 316
- FR-M- 805
- GB-A- 929 252
- CHEMICAL ABSTRACTS, vol. 79, 6 août 1973, page 462, abrégé no. 32098k,Columbus, Ohio, US;

## Description

La présente invention concerne un nouveau procédé industriel de préparation de la 1-(2,3,4-triméthoxybenzyl) pipérazine.

Ce composé connu aussi sous la DCI trimétazidine, possède des propriétés pharmacologiques très intéressantes. Il est doué notamment des propriétés vasodilatatrices et préserve les métabolismes énergétiques de la cellule exposée à l'hypoxie ou à l'ischémie et évite l'effondrement du taux intracellulaire de l'ATP.

Plusieurs méthodes de préparation de ce composé sont déjà connues. Toutefois, aucun procédé déjà décrit dans la littérature ne permet l'obtention de la trimétazidine avec une pureté satisfaisante, et avec un bon rendement.

Le procédé de préparation de la 1-(2,3,4-triméthoxybenzyl) piperazine préconisé dans le BSM N° 805 M, consiste à obtenir ce composé en condensant le chlorure du 2,3,4-triméthoxybenzyle avec la 1-formyl piperazine. Ensuite, le produit de la condensation est hydrolysé pour obtenir la 1-(2,3,4-triméthoxybenzyl) pipérazine, que l'on traite par l'acide chlorhydrique gazeux pour obtenir le dichlorohydrate correspondant.

Ce procédé fournit avec un rendement peu satisfaisant une matière première de pureté moyenne. Plusieurs purifications sont donc nécessaires pour obtenir un produit de "qualité pharmaceutique".

On a décrit par ailleurs dans le Brevet Français N° 1.302958 différents procédés de préparation des dérivés trialcoxylés de la 1-benzylpipérazine, et en particulier de la 1-(2,3,4-triméthoxybenzyl) pipérazine.

Toutefois, tous ces procédés nécessitent généralement plusieurs étapes et permettent l'obtention de la trimétazidine avec des rendements qui ne dépassent pas 43 %.

Les procédés décrits conduisent aussi à la formation d'un grand nombre de produits secondaires.

Un autre procédé de préparation de la trimétazidine est décrit dans le Brevet Français N° 2493316. Ce procédé consiste à faire réagir dans un premier solvant, le chlorure de 2,3,4-triméthoxybenzyle sur la pipérazin-2-one, pour obtenir la 4-(2,3,4-triméthoxybenzyl)-pipérazin-2-one puis dans une deuxième phase, à réduire ce composé dans une second solvant, au moyen d'un hydrure, pour obtenir le composé attendu. Ce procédé nécessite la préparation de la pipérazin-2-one, produit non commercial.

Le Brevet JP 48032889 décrit un procédé de préparation de la trimétazidine, à partir du 2,3,4-triméthoxy-benzylaldéhyde et de la pipérazine hexahydratée. La réaction est réalisée à 80-90°C en présence de l'acide formique pendant 10-18 heures, et le rendement est de l'ordre de 38 %.

Compte-tenu de l'intérêt thérapeutique de la trimétazidine, et l'absence d'un procédé permettant son obtention avec un bon rendement, une pureté satisfaisante et si possible à partir des matières premières peu onéreuses et disponibles dans le commerce, des recherches plus approfondies ont été entreprises, et ont abouti à la découverte d'un nouveau procédé de préparation de la 1-(2,3,4-triméthoxybenzyl) pipérazine et du dichlorohydrate correspondant.

Ce procédé permet l'obtention de la trimétazidine base en une étape à partir des composés commerciaux, avec un rendement supérieur à 90 % et avec une pureté très satisfaisante.

L'invention a plus précisément pour objet un procédé de préparation de la 1-(2,3,4-triméthoxybenzyl) pipérazine, composé de formule I : 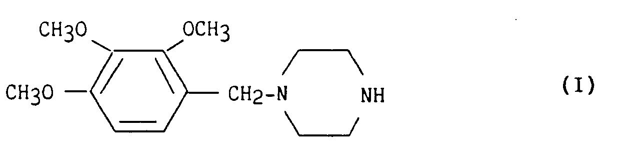
caractérisé en ce que l'on met en solution le 2,3,4-triméthoxy benzaldéhyde, composé de formule II : 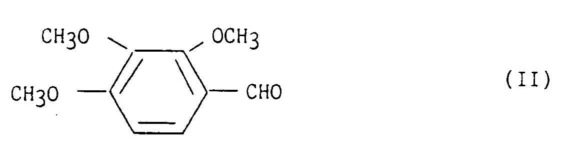
avec la pipérazine, composé de fomule III : 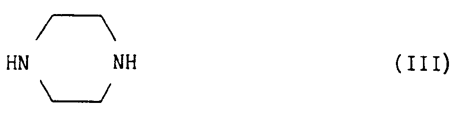
dans un solvant alcoolique ou dans le méthyl-tertiobutyl-éther (MTBE), puis on soumet la solution ainsi obtenue à l'action de l'hydrogène, en présence d'un catalyseur d'hydrogénation, et à une température comprise entre 45 et 75°C, pour former le composé de formule I,
puis on sépare du milieu réactionnel la pipérazine en excès, et ensuite le composé de formule I sous forme de base,
lequel ensuite si on le désire,
est transformé en ses sels d'addition avec un acide organique ou minéral pharmaceutiquement acceptable.

Les matières premières de départ, 2,3,4-triméthoxybenzaldéhyde et pipérazine, sont des produits commerciaux.
(2,3,4-triméthoxybenzaldéhyde fourni par FINORGA® et pipérazine anhydre fournie par BASF ®)

Lors de la réaction, les composés de formule II et III, sont mis en solution, soit dans un alcool de petit poids moléculaire tels que l'éthanol ou l'isopropanol, soit d'une manière préférentielle, dans le M.T.B.E.

Comme catalyseur d'hydrogénation, on peut utiliser le Pd/C à 5 % ou le Pt/C à 5 %. De manière préférentielle on utilise le Pd/C à 5 %.

Lors de la réaction, un excés de pipérazine est nécessaire. En effet, le rapport molaire 2,3,4-triméthoxybenzaldéhyde et pipérazine doit être égal à 1:2 -1:4.

Lors de l'hydrogénation, la température au début peut-être fixée à 45-55°C. Elle se stabilise ensuite en cours d'hydrogénation vers 70-75°C. La pression de l'hydrogène est fixée de manière préférentielle entre 5 et 10 bars.

Quand le M.T.B.E est utilisé comme solvant, l'excès de la pipérazine est isolé à la fin de la réaction, après refroidissement du milieu réactionnel, par simple précipitation.

La pipérazine ainsi isolée, à l'état pur, peut être utilisée ultérieurement. Concernant la trimétazidine, elle est isolée du milieu réactionnel par double extraction.

En effet, après élimination de la pipérazine, et ajustement du pH du milieu réactionnel à 7,5 - 8,3 la trimétazidine est extraite par l'eau puis, après alcalinisation, par le toluène.

Les exemples suivants illustrent l'invention.

### EXEMPLE 1 :

### Préparation de la trimétazidine par amination réductive en utilisant comme solvant réactionnel le M.T.B.E

Dans un réacteur, charger 78,4 g de 2,3,4-triméthoxybenzaldéhyde, 68,8 g de pipérazine, 400 ml de M.T.B.E. et 4 g de Pd/C à 5 % (Engelhard®).

Purger à l'azote et à l'hydrogène, puis chauffer le plus rapidement possible.

Hydrogener sous 10 bars, dès que la température atteint environ 50-55°C et continuer à chauffer jusqu'à 70°C.

Arrêter l'hydrogénation après environ 2 heures. Refroidir à 50°C et filtrer pour éliminer le catalyseur.

Récupérer le filtrat et refroidir à environ 10°C pour éliminer par précipitation la pipérazine qui n'a pas réagi. Filtrer.

A 13-18°C, ajouter au filtrat 200 ml d'eau et ajuster le pH entre 7,9 et 8,0, par addition d'acide chlorhydrique 7 N.

Diluer ensuite au demi avec de l'eau.

Eliminer la phase organique et extraire la phase aqueuse 2 fois avec 100 ml de toluène. Eliminer le toluène.

Alcaliniser ensuite la phase aqueuse, en refroidissant sur bain de glace, par addition de 42 g de soude en pastilles.

Extraire la trimétazidine base ainsi relarguée, 3 fois avec 120 ml de toluène. Sécher les extraits toluèniques sur sulfate de magnésium anhydre et évaporer à sec.
Rendement : 94 %
Pureté de la trimétazidine (CLHP) : 99,5 %

### EXEMPLE 2

### Préparation de la trimétazidine par amination réductive en utilisant comme solvant réactionnel l'éthanol

Dans un réacteur charger 78,4 g de 2,3,4-triméthoxybenzaldéhyde, 137,6 g de pipérazine anhydre, 400 ml d'éthanol et 4 g de Pd/C à 5 %.

Purger à l'azote et à l'hydrogène, puis chauffer le plus rapidement possible à 70°C. Hydrogéner sous 10 bars dès que la température atteint 50°C. Arrêter l'hydrogénation après 70 min.

Filter le milieu réactionnel à 20°C et récupérer le filtrat jaune limpide. Evaporer à sec.

Reprendre le résidu pâteux dans 200 ml de toluène glacé entre -5 et -10°C.

Eliminer la pipérazine précipitée par filtration.

Ajouter 200 ml d'eau au filtrat toluènique et amener le pH à 6 par addition d'acide chlorhydrique concentré. Décanter, et extraire deux fois encore avec 120 ml de toluène.

Eliminer les phases organiques puis alcaliniser avec 42 g de soude en paillette. La trimétazidine relargue.

Extraire trois fois avec 120 ml de toluène. Réunir les phases toluèniques puis évaporer à sec pour isoler la trimétazidine base.
Rendement : 92 %
Pureté de la trimétazidine (CLHP) : 96,8 %

### EXEMPLE 3

### Préparation du dichlorhydrate de la trimétazidine

Dans un réacteur mis sous atmosphère d'azote, charger sous agitation 216,0 g d'isopropanol et 100,1 g de trimétazidine base obtenue dans l'exemple 1.

Agiter jusqu'à dissolution totale de la base.

Filtrer et recueillir la solution limpide de trimétazidine base dans un container inox. Rincer le réacteur avec 15,7 g d'isopropanol et mélanger l'isopropanol de rinçage avec la solution de la trimétazidine.

Dans un réacteur, charger sous atmosphère d'azote 348 g d'isopropanol, et 79,2 g d'acide chlorhydrique concentré (36 %).

Couler alors sur cette solution sous agitation, sans dépasser 40°C, la solution de la trimétazidine base.

Le milieu est ensuite concentré à pression ordinaire jusqu'à obtention d'un poids d'évaporat de 270 g.

La suspension ainsi obtenue est refroidie à 0°C et maintenue à cette température pendant 1 heure sous agitation et sous azote.

Filtrer ensuite la suspension pour isoler le dichlorhydrate de la trimétazidine. Ce sel est ensuite lavé deux fois par l'isopropanol.
Rendement : 99 %
Pureté du chlorhydrate de trimétazidine (CLHP) : 100 %

## Revendications (Revendications pour l'(les) Etat(s) contractant(s) suivant(s): AT, BE, CH, DE, DK, FR, GB, IT, LI, LU, NL, SE)

1. Procédé de préparation de la 1-(2,3,4-triméthoxybenzyl) pipérazine, composé de formule I : 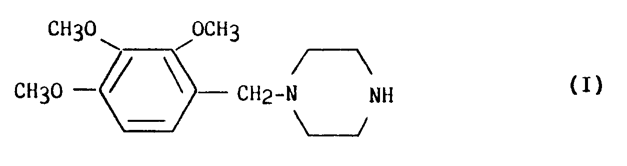 caractérisé en ce que l'on met en solution le 2,3,4-triméthoxybenzaldéhyde, composé de formule II : 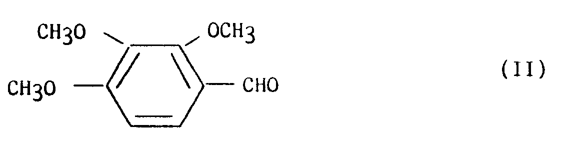 avec un excès de piperazine, composé de fomule III : 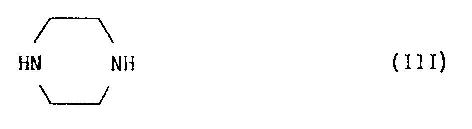 (l'excès étant de 2 à 4 moles de composé III pour 1 mole de composé II)
dans un solvant alcoolique ou dans le méthyl-tertiobutyl-éther, puis, on soumet la solution ainsi obtenue à l'action de l'hydrogène, en présence d'un catalyseur d'hydrogénation, à une température de 45 à 55 °C au début qui se stabilise ensuite vers 70-75 °C, et sous une pression d'hydrogène entre 5 et 10 bars pour former le composé de formule I,
puis on sépare du milieu réactionnel la pipérazine en excès, et ensuite le composé de formule I sous forme de base,
lequel ensuite, si on le désire,
est transformé en ses sels d'addition avec un acide organique ou minéral pharmaceutiquement acceptable.

2. Procédé selon la revendication 1 caractérisé en ce que les composés de formule II et III sont mis en solution dans le méthyl-tertiobutyl-éther.

3. Procédé selon la revendication 1 caractérisé en ce que le palladium sur charbon à 5 % est utilisé comme catalyseur d'hydrogénation.

4. Procédé selon la revendication 1 caractérisé en ce que le rapport molaire du 2,3,4-triméthoxybenzaldéhyde et de la pipérazine est 1:2.

5. Procédé selon la revendication 1 caractérisé en ce que les composés de formule II et III sont mis en solution dans le méthyl-tertiobutyl-éther et que l'excès de la pipérazine après la réaction est éliminée du milieu réactionnel par précipitation.

## Revendications (Revendications pour l'(les) Etat(s) contractant(s) suivant(s): ES, GR)

1. Procédé de préparation de la 1-(2,3,4-triméthoxybenzyl) pipérazine, composé de formule I : 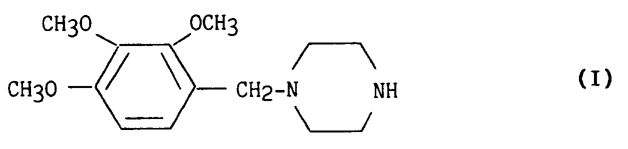 caractérisé en ce que l'on met en solution le 2,3,4-triméthoxybenzaldéhyde, composé de formule II : 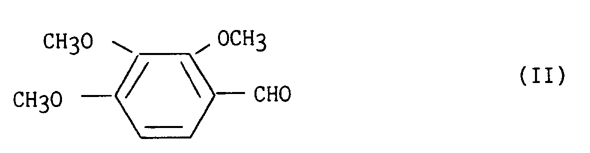 avec un excès de piperazine, composé de fomule III : 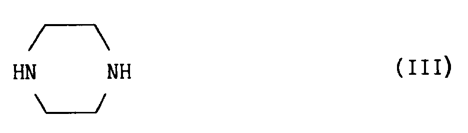 (l'excès étant de 2 à 4 moles de composé III pour 1 mole de composé II)
dans un solvant alcoolique ou dans le méthyl-tertiobutyl-éther, puis, on soumet la solution ainsi obtenue à l'action de l'hydrogène, en présence d'un catalyseur d'hydrogénation, à une température de 45 à 55 °C au début qui se stabilise ensuite vers 70-75 °C, et sous une pression d'hydrogène entre 5 et 10 bars pour former le composé de formule I,
puis on sépare du milieu réactionnel la pipérazine en excès, et ensuite le composé de formule I sous forme de base,
lequel ensuite, si on le désire,
est transformé en ses sels d'addition avec un acide organique ou minéral pharmaceutiquement acceptable.

2. Procédé selon la revendication 1 caractérisé en ce que les composés de formule II et III sont mis en solution dans le méthyl-tertiobutyl-éther.

3. Procédé selon la revendication 1 caractérisé en ce que le palladium sur charbon à 5 % est utilisé comme catalyseur d'hydrogénation.

4. Procédé selon la revendication 1 caractérisé en ce que le rapport molaire du 2,3,4-triméthoxybenzaldéhyde et de la pipérazine est 1:2.

5. Procédé selon la revendication 1 caractérisé en ce que les composés de formule II et III sont mis en solution dans le méthyl-tertiobutyl-éther et que l'excès de la pipérazine après la réaction est éliminée du milieu réactionnel par précipitation.

## Claims (Claims for the following Contracting State(s): AT, BE, CH, DE, DK, FR, GB, IT, LI, LU, NL, SE)

1. Process for the preparation of 1-(2,3,4-trimethoxybenzyl)piperazine, the compound of formula I: 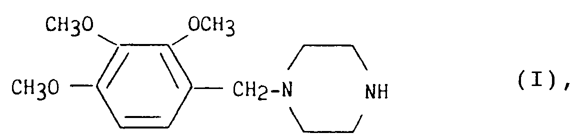 characterised in that 2,3,4-trimethoxybenzaldehyde, the compound of formula II: 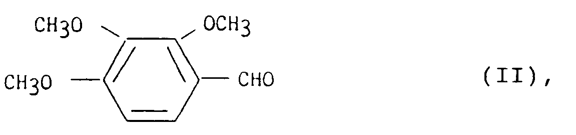 is dissolved with an excess of piperazine, the compound of formula III: 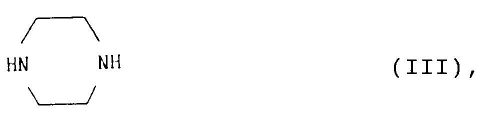 (the excess being from 2 to 4 moles of compound III per mole of compound II)
in an alcoholic solvent or in methyl tert.-butyl ether, the solution so obtained is then subjected to the action of hydrogen, in the presence of a hydrogenation catalyst, at a temperature which is at first from 45 to 55°C and which then stabilises at about 70-75°C, and under a hydrogen pressure of from 5 to 10 bar, to give the compound of formula I,
there are then separated from the reaction medium first the excess piperazine, and then the compound of formula I in base form,
which compound, if desired, is then converted into its addition salts with a pharmaceutically acceptable organic or mineral acid.

2. Process according to claim 1, characterised in that the compounds of formulae II and III are dissolved in methyl tert.-butyl ether.

3. Process according to claim 1, characterised in that 5% palladium-on-carbon is used as hydrogenation catalyst.

4. Process according to claim 1, characterised in that the molar ratio of 2,3,4-trimethoxybenzaldehyde to piperazine is 1:2.

5. Process according to claim 1, characterised in that the compounds of formulae II and III are dissolved in methyl tert.-butyl ether and in that the excess piperazine, after the reaction, is removed from the reaction medium by precipitation.

## Claims (Claims for the following Contracting State(s): ES, GR)

1. Process for the preparation of 1-(2,3,4-trimethoxybenzyl)piperazine, the compound of formula I: 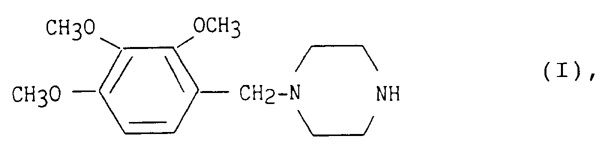 characterised in that 2,3,4-trimethoxybenzaldehyde, the compound of formula II: 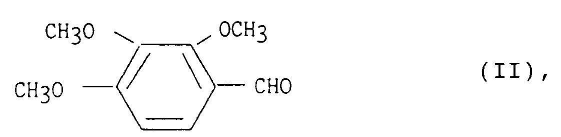 is dissolved with an excess of piperazine, the compound of formula III: 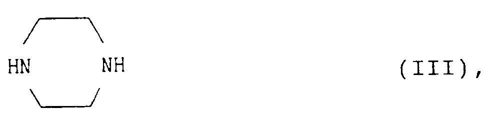 (the excess being from 2 to 4 moles of compound III per mole of compound II)
in an alcoholic solvent or in methyl tert.-butyl ether, the solution so obtained is then subjected to the action of hydrogen, in the presence of a hydrogenation catalyst, at a temperature which is at first from 45 to 55°C and which then stabilises at about 70-75°C, and under a hydrogen pressure of from 5 to 10 bar, to give the compound of formula I,
there are then separated from the reaction medium first the excess piperazine, and then the compound of formula I in base form,
which compound, if desired, is then converted into its addition salts with a pharmaceutically acceptable organic or mineral acid.

2. Process according to claim 1, characterised in that the compounds of formulae II and III are dissolved in methyl tert.-butyl ether.

3. Process according to claim 1, characterised in that 5% palladium-on-carbon is used as hydrogenation catalyst.

4. Process according to claim 1, characterised in that the molar ratio of 2,3,4-trimethoxybenzaldehyde to piperazine is 1:2.

5. Process according to claim 1, characterised in that the compounds of formulae II and III are dissolved in methyl tert.-butyl ether and in that the excess piperazine, after the reaction, is removed from the reaction medium by precipitation.

## Patentansprüche (Patentansprüche für folgende(n) Vertragsstaat(en): AT, BE, CH, DE, DK, FR, GB, IT, LI, LU, NL, SE)

1. Verfahren zur Herstellung von 1-(2,3,4-Trimethoxybenzyl)-piperazin der Formel I: 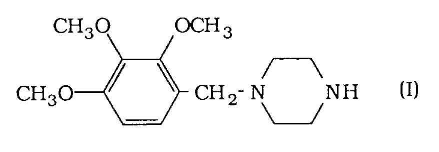 **dadurch gekennzeichnet**, daß man 2,3,4-Trimethoxybenzaldehyd der Formel II: 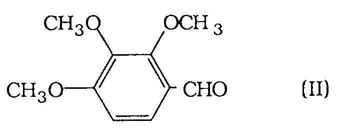 mit einem Überschuß von Piperazin der Formel III: 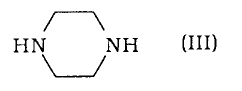 (wobei der Überschuß 2 bis 4 Mol der Verbindung III pro Mol der Verbindung II beträgt)
in einem alkoholischen Lösungsmittel oder in Methyl-tert.-butylether löst und die in dieser Weise erhaltene Lösung in Gegenwart eines Hydrierkatalysators bei einer Anfangstemperatur von 45 bis 55°C, die sich anschließend bei 70 - 75°C stabilisiert, und bei einem Wasserstoffdruck zwischen 5 und 10 bar der Einwirkung von Wasserstoff aussetzt zur Bildung der Verbindung der Formel I,
dann aus dem Reaktionsmedium das überschüssige Piperazin und anschließend die Verbindung der Formel I in Form der Base entfernt,
welche man anschließend gewünschtenfalls
mit einer organischen oder anorganischen, pharmazeutisch annehmbaren Säure in ihre Additionssalze umwandelt.

2. Verfahren nach Anspruch 1, **dadurch gekennzeichnet**, daß man die Verbindungen der Formel II und III in Methyl-tert.-butylether löst.

3. Verfahren nach Anspruch 1, **dadurch gekennzeichnet**, daß man als Hydrierkatalysator 5 % Palladium-auf-Kohlenstoff verwendet.

4. Verfahren nach Anspruch 1, **dadurch gekennzeichnet**, daß das Molverhältnis von 2,3,4-Trimethoxybenzaldehyd zu Piperazin 1:2 beträgt.

5. Verfahren nach Anspruch 1, **dadurch gekennzeichnet**, daß man die Verbindungen der Formel II und III in Methyl-tert.-butylether löst und nach der Reaktion das überschüssige Piperazin durch Ausfällen aus dem Reaktionsmedium entfernt.

## Patentansprüche (Patentansprüche für folgende(n) Vertragsstaat(en): ES, GR)

1. Verfahren zur Herstellung von 1-(2,3,4-Trimethoxybenzyl)-piperazin der Formel I: 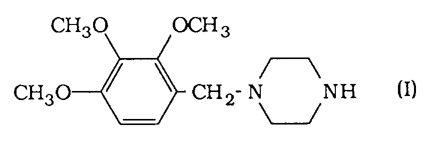 **dadurch gekennzeichnet**, daß man 2,3,4-Trimethoxybenzaldehyd der Formel II: 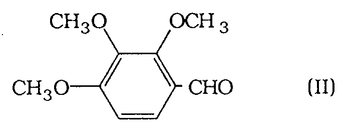 mit einem Überschuß von Piperazin der Formel III: 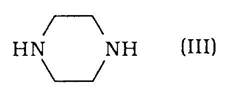 (wobei der Überschuß 2 bis 4 Mol der Verbindung III pro Mol der Verbindung II beträgt)
in einem alkoholischen Lösungsmittel oder in Methyl-tert.-butylether löst und die in dieser Weise erhaltene Lösung in Gegenwart eines Hydrierkatalysators bei einer Anfangstemperatur von 45 bis 55°C, die sich anschließend bei 70 - 75°C stabilisiert, und bei einem Wasserstoffdruck zwischen 5 und 10 bar der Einwirkung von Wasserstoff aussetzt zur Bildung der Verbindung der Formel I,
dann aus dem Reaktionsmedium das überschüssige Piperazin und anschließend die Verbindung der Formel I in Form der Base entfernt,
welche man anschließend gewünschtenfalls
mit einer organischen oder anorganischen, pharmazeutisch annehmbaren Säure in ihre Additionssalze umwandelt.

2. Verfahren nach Anspruch 1, **dadurch gekennzeichnet**, daß man die Verbindungen der Formel II und III in Methyl-tert.-butylether löst.

3. Verfahren nach Anspruch 1, **dadurch gekennzeichnet**, daß man als Hydrierkatalysator 5 % Palladium-auf-Kohlenstoff verwendet.

4. Verfahren nach Anspruch 1, **dadurch gekennzeichnet**, daß das Molverhältnis von 2,3,4-Trimethoxybenzaldehyd zu Piperazin 1:2 beträgt.

5. Verfahren nach Anspruch 1, **dadurch gekennzeichnet**, daß man die Verbindungen der Formel II und III in Methyl-tert.-butylether löst und nach der Reaktion das überschüssige Piperazin durch Ausfällen aus dem Reaktionsmedium entfernt.
